# EUROPEAN PATENT SPECIFICATION

(11) **EP 1 601 322 B1**
(45) Date of publication and mention of the grant of the patent: **03.12.2008**
(21) Application number: 04719395.8
(22) Date of filing: 11.03.2004
(51) Int. Cl.: A61F 15/00

(54) **PACKAGED FILM-COVERED TAMPON**
VERPACKTER FILMBESCHICHTETER TAMPON
TAMPON REVETU D'UN FILM EMBALLE

(30) Priority: 11.03.2003 US 505351 P
(43) Date of publication of application: 07.12.2005
(73) Proprietor: JOHNSON & JOHNSON GmbH, 40474 Düsseldorf (DE)
(72) Inventor: SCHOELLING, Hans-Werner, 58242 Ennepetal (DE); JANSSEN, William, 9651 CJ Meeden (NL); WEITZ, Simone, 44149 Dortmund (DE); ARBECK, Yvonne, 40476 Düsseldorf (DE); BRINKMANN, Ute, 64319 Pfungstadt (DE); BUSKOTTE, Ulrich, 42897 Remscheid (DE)
(74) Representative: Metten, Karl-Heinz
(86) International application number: PCT/EP2004/002530
(87) International publication number: WO 2004/080362

(56) References cited:
- WO-A-99/07553
- DE-A- 10 109 608
- US-A- 4 610 659
- PATENT ABSTRACTS OF JAPAN vol. 1998, no. 08, 30 June 1998 (1998-06-30) & JP 10 072057 A (NITTO DENKO CORP), 17 March 1998 (1998-03-17)

## Description

### Field of the Invention

The present invention relates to a packaged apertured film-covered tampon. In particular, the invention relates to a combination of apertured film-covered tampon and an overwrap package that has acceptable friction characteristics between the two elements to maintain easy removal of the tampon from the package.

### Background of the Invention

A covered, spirally wound tampon is disclosed in Friese, US Pat. No. 4,816,100. This tampon has a liquid-permeable, thermoplastic strip section bonded by heat-sealing to the outside of the nonwoven web section. The outer end of the strip section, which projects beyond the end of the nonwoven web section, is bonded to the outside of part of the strip section sealed to the nonwoven web section. Both the insertion and withdrawal end of the tampon remain free of the cover material.

Recently, there has been interest in replacing nonwoven tampon covers with apertured polymeric film covers, such as disclosed in Hull et al., WO 01/02144 "Continuous Method of Providing Individual Sheets from a Continuous Web". However, in trying to commercialize these, it becomes apparent that the overwrap package removal is too difficult using conventional packages such as described in Warncke, US Pat. No. 4,583,964.

Therefore, what is needed is tampon overwrap package that the user can remove from the enclosed tampon without difficulty. What is needed is an apertured, polymeric film-covered tampon and overwrap package system that requires low shear forces to remove the overwrap package from the tampon.

### Summary of the Invention

Accordingly, a packaged tampon according to the features of claim 1 has been found. Thereby, the shear force necessary to remove the tampon from its package is reduced to less than about 5 N after conditioning at 40°C / 75% humidity for one week.

In one aspect of the invention, the overwrap package film is embossed in the form of a regular array of pyramids having a height of less than about 100 micrometers.

In another aspect of the invention, a solid coating is applied to either the overwrap package film or the apertured film tampon cover, through techniques including vapor deposition and sputtering.

Other aspects and features of the present invention will become apparent in those ordinarily skilled in the art upon review of the following description of specific embodiments of the invention in conjunction with the accompanying drawings.

### Brief Description of the Drawing

Fig. 1 is a partially broken-away perspective view of a tampon according to the present invention;
Fig. 1A is a graphical representation of the data presented in Table 1;
Fig. 2 is a graphical representation of the data presented in Table 2;
Fig. 3 is a graphical representation of the data presented in Table 3;
Fig. 4 is a graphical representation of the data presented in Table 4.

### Detailed Description of the Preferred Embodiments

Absorbent tampons usually incorporate elongate compressed absorbent structures, such as substantially cylindrical masses of compressed absorbent material having a central axis and a radius that defines the outer circumferential surface of the tampon. Tampons are often formed by first obtaining a shaped mass of absorbent material called a tampon blank. This blank can be in the form of a roll of sheet-like material, a segment of a continuous absorbent material, a mass of randomly or substantially uniformly oriented absorbent material, an individually prepared or cast mass of absorbent material, and the like.

The tampon blank is relatively uncompressed and has a relatively low density. It is then compressed to form a product having overall dimensions less than those of the blank prior to use. The compressed tampons may have a generally uniform density throughout the tampon, or they may have regions of differing density as described in the commonly assigned applications to Friese et al., US Pat. No. 6,310,269, and Leutwyler et al., US Pat. No. 5,911,712,. Tampons also usually include a cover or some other surface treatment and a withdrawal string or other removal mechanism.

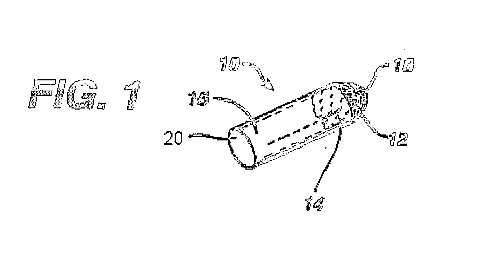

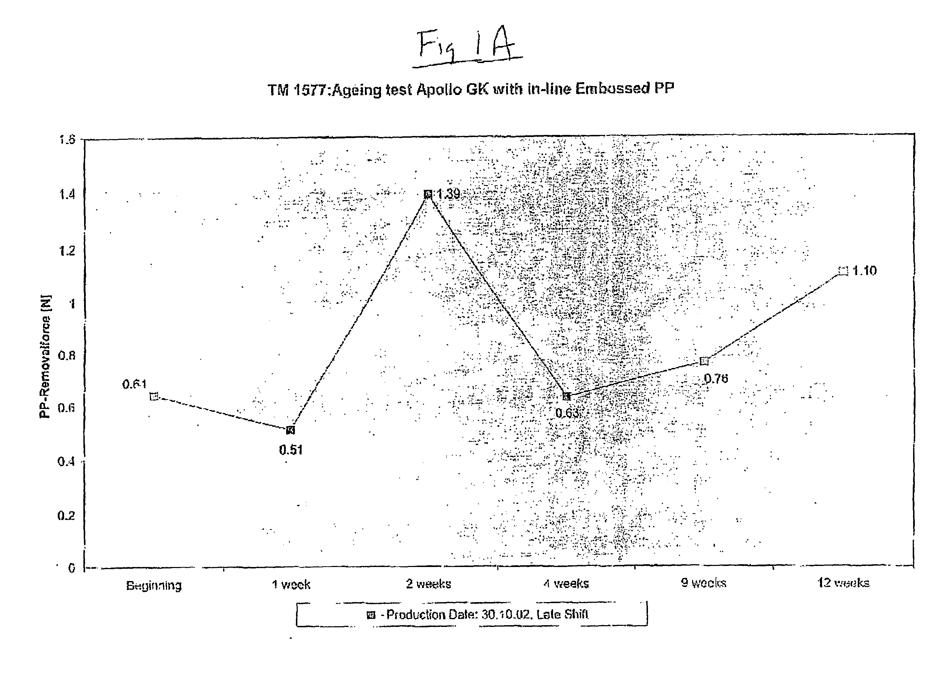

An example of a packaged tampon useful for feminine hygiene according to the present invention is illustrated in Fig. 1. This tampon 10 has a compressed, elongate absorbent structure 12 substantially surrounded by an apertured polymeric film cover 14. The compressed tampon 10 is packaged in an overwrap package 16 comprising a polymeric film in contact with the apertured polymeric film cover 14 and containing the tampon 10 under compression. The overwrap package 16 is removable from the compressed tampon 10 by sliding it along at least a portion of a length of the tampon 10 by applying a shear force of less than about 5 Newtons between the package 16 and the tampon 10.

Absorbent materials useful in the formation of the absorbent body include fiber, foam, superabsorbent, hydrogels, and the like. Preferred absorbent material for the present invention includes foam and fiber. Absorbent foams may include hydrophilic foams, foams which are readily wetted by aqueous fluids as well as foams in which the cell walls that form the foam themselves absorb fluid.

Preferably, the fibers employed in the formation of the absorbent body include regenerated cellulosic fiber, natural fibers and synthetic fibers. Preferably, the materials employed in the formation of a tampon according to the present invention include fiber, foam, hydrogels, wood pulp, superabsorbents, and the like. A useful, non-limiting list of useful absorbent body fibers includes natural fibers such as cotton, wood pulp, jute, and the like; and processed fibers such as regenerated cellulose, cellulose nitrate, cellulose acetate, rayon, polyester, polyvinyl alcohol, polyolefin, polyamine, polyamide, polyacrylonitrile, and the like. Other fibers in addition to the above fibers may be included to add desirable characteristics to the absorbent body. Preferably, tampon fibers are rayon or cotton, and more preferably, the fibers are rayon. The fibers may have any useful cross-section.

Fiber cross-sections include multi-limbed and non-limbed. Multi-limbed, regenerated cellulosic fibers have been commercially available for a number of years. These fibers are known to possess increased specific absorbency over non-limbed fibers. A commercial example of these fibers is the Danufil® VY multilimbed viscose rayon fibers available from Acordis UK Ltd., Spondon, England. These fibers are described in detail in Wilkes et al., US Pat. No. 5,458,835, the disclosure of which is hereby incorporated by reference. Preferably, the fibers include hydrophilic fibers, and more preferably, the fibers include absorbent fibers, i.e., the individual fibers, themselves, absorb fluid. A useful, non-limiting list of useful tampon fibers includes natural fibers such as cotton, wood pulp, jute, hemp, and the like; and processed fibers such as regenerated cellulose, cellulose nitrate, cellulose acetate, rayon, polyester, polyvinyl alcohol, polyolefin, polyamine, polyamide, polyacrylonitrile, and the like. Other fibers in addition to the above fibers may be included to add desirable characteristics to the absorbent body. For example, hydrophobic fibers may be used in outer surfaces of the tampon to reduce surface wetness and hydrophilic fibers may be used to increase the rate of fluid transport into and throughout the body. Preferably, the tampon fibers are rayon or cotton, and more preferably, the fibers are rayon. The fibers may have any useful cross-section.

The tampon blank is substantially surrounded or enclosed by a fluid-permeable, apertured polymeric film cover 14. Thus, the cover 14 encloses a majority of the outer surface of the tampon 10. This may be achieved as disclosed in Friese, U.S. Patent No. 4,816,100, or Lochte et al., US Serial No. 09/607032, entitled "Tampon Having Apertured Film Cover Thermobonded to Fibrous Absorbent Structure", filed June 29, 2000 (Atty Docket J&J-1925), . In addition, the insertion end 18 of the tampon, the opposite withdrawal end 20, or both may be enclosed by the cover. Of course, for processing or other reasons, some portions of the surface of the tampon 10 may be free of the cover 14. For example, the insertion end 18 of the tampon 10 and a portion of the cylindrical surface adjacent this end may be exposed, without the cover 14 to allow the tampon 10 to more readily accept fluids.

The apertured polymeric film cover 14 can ease the insertion of the tampon 10 into the body cavity and can reduce the possibility of fibers being separated from the tampon 10. Apertured polymeric films useful in forming the cover are known to those of ordinary skill in the art.

When pressure is released after moderate mechanical compression, a tampon tends to expand toward its original dimensions. Therefore, tampon blanks are generally overcompressed to allow them to rebound slightly to the desired density for use. Over-compression mechanically constricts expansion to prevent the tampon from expanding without added liquid.

Tampons are generally categorized in two classes: applicator tampons and digital tampons, and a certain amount of dimensional stability is useful for each type of tampon. Applicator tampons use a relatively rigid device to contain and protect the tampon prior to use. To insert the tampon into a body cavity, the applicator is partially inserted into the body cavity, and the tampon can be expelled therefrom. In contrast, digital tampons do not have an applicator to help guide them into the body cavity and require sufficient column strength to allow insertion without using an applicator. This strength can be determined by securing one end of the tampon to the fixed plate of a Instron Universal Testing Machine, available from Instron Corporation, Canton, Massachusetts, USA. The moveable plate is brought to contact the opposite end of the tampon and is then set to compress the tampon at a rate of about 5 cm/minute. The force exerted on the tampon is measured continuously, and the point at which this force begins to fall instead of rise is the point at which the tampon buckles. The maximum force achieved is the tampon stability.

As indicated above, overcompression of the tampon 10 allows them to rebound slightly to the desired density for use. Some of this rebound occurs after the tampon is placed into the overwrap package. Rebound of the packaged tampon creates pressure between the tampon and the overwrap package. Commercial overwrap packages generally have a tear strip located near the middle of the product, and one or more portions of the package are then slid off the ends. The pressure resulting from the tampon rebound can make this sliding removal more difficult.

Again, the overwrap package 16 is removable from the compressed tampon 10 by sliding it along at least a portion of a length of the tampon 10 by applying a shear force. We have found that conventional overwrap packages formed of standard cellophane or polypropylene films do not allow the tampon and package to slide easily against each other. This increases the difficulty of removing the tampon from its package, and it would increase the user's frustrations. Therefore, we have found it necessary to reduce the shear force necessary to remove the tampon from its package to less than about 5 N after conditioning at 40 °C / 75 % humidity for one week, preferably, less than about 3 N, and most preferably about 2 N after such conditioning. The tampon may be conditioned immediately after its production, or it may be conditioned after storage, e.g., after purchase from a retail outlet.
The overwrap removal force is reduced by embossing (including microembossing) or electronically altering (including corona discharge treatments) the film from which the overwrap and/ or tampon cover are formed.

The embossing may be formed on the film using conventional embossing techniques. The pattern embossed on the overwrap package film can take the form of a regular array of pyramids having a height of less than about 100 micrometers, more preferably, less than about 50 micrometers, and most preferably, less than about 25 micrometers. The pyramids can be formed by cutting two groups of parallel v-shaped grooves into the surface of the embossing roller. The first group of grooves is angled with respect to the second group. Preferably, the angle between groups is near 90 degrees, but other angles can be used, depending upon desired film characteristics.

The spacing of the grooves can be measured by the number of grooves per centimeter. Preferably, there are between about 20 and 100 grooves per centimeter, more preferably, between about 40 and 80 grooves per centimeter, and most preferably, between about 55 and 65 grooves per centimeter.

Solid coatings may be applied to one of the films through techniques including vapor deposition and sputtering. In addition, slip agents such as fluid lubricants or solid layers with a reduced coefficient of friction may be applied to the tampon cover at any appropriate portion of the manufacturing process.

The overwrap package may be formed in conventional manners, such as are described in Warncke, US Pat. No. 4,583,964, and Lewis et al., US Serial No. 09/204696, entitled "Tampon for Feminine Hygiene or Medical Purposes, and Process for Producing the Same", filed December 3, 1998 (Atty Docket J&J1693),.

### Examples

The present invention will be further understood by reference to the following specific Examples that are illustrative of the composition, form and method of producing the device of the present invention. It is to be understood that many variations of composition, form and method of producing the device would be apparent to those skilled in the art. The following Examples, wherein parts and percentages are by weight unless otherwise indicated, are only illustrative.

### 1. Purpose

To determine the necessary force to remove the polypropylene wrapper from the tampon's string end.

### 2. Principle of Method

The polypropylene wrapper at the tampon's string end is removed from the tampon by a tractive force. The applied force to remove the wrapper is measured.

### 3. Analysis Time

Each measurement (20 tampons) will take ca. 30 minutes.

### 4. Apparatus

• Instron machine 5543 with 50 N -load cell and pneumatic clamps (width: 50 mm), computer-controlled.
• Windows 95/98 (Microsoft)
• Merlin Software (Instron) to operate Instron machine and display the results
• printer

### 5. Preparation

5.1. Remove tear strip and overwrap package covering the insertion end from the tampons.

5.2. An adhesive tape (brand: Tesa) is wound around the polypropylene wrapper at the string end in such a manner that the adhesive strip forms a longitudinal extension of the tampon.

### 6. Execution of Measurement

6.1. Insert the tampon into the testing device. The insertion end of the tampon is fixed vertically into the lower clamps. The adhesive strip is locked between the upper clamps.

6.2. Start the measurement by setting the test machine in the operating mode with a crosshead velocity of 100 mm/min. The test stops after an extension of 10 mm is reached. The maximum applied shear force is recorded in Newtons (N).

### 7. Annex

7.1 Accuracy of measurement

Speed accuracy: ± 0.1 % of set speed

Extension accuracy: The greater of ± 0.02 mm or 0.05 %

Load accuracy (0.4 - 1.0 % of cell capacity resp. readings between 0.2 - 0.5 N): ± 1,0 % of reading (< 0.005 N)

Load accuracy (1.0 - 10.0 of cell capacity resp. readings between 0.5 - 5.0 N): ± 0,5 % of reading (< 0.025 N)

Load accuracy (10.0 - 100.0 % of cell capacity resp. readings between 5.0 - 50.0 N): ± 0,4 % of reading (< 0.2 N)

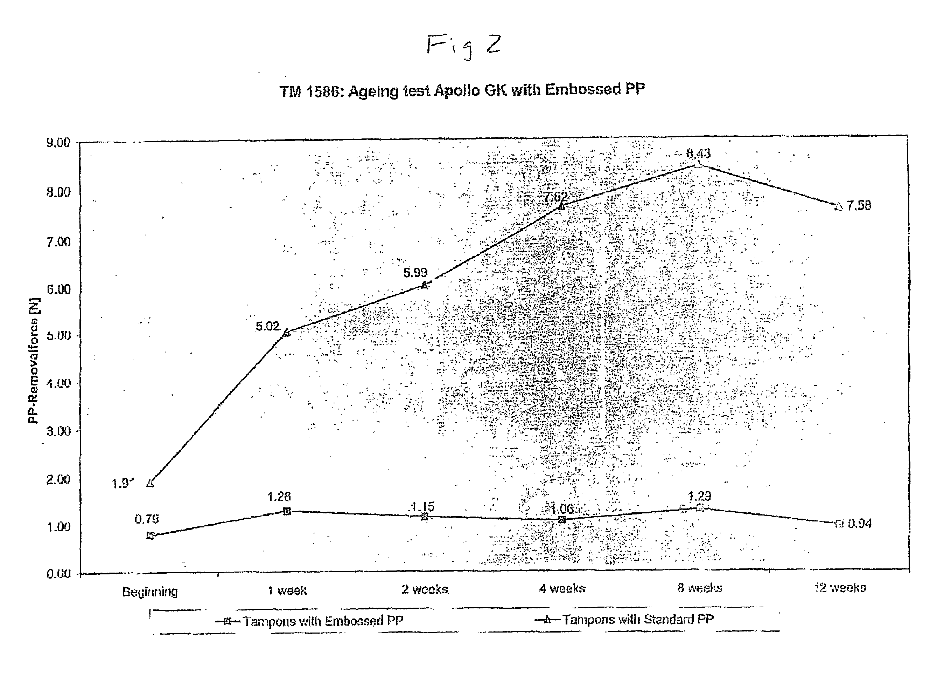

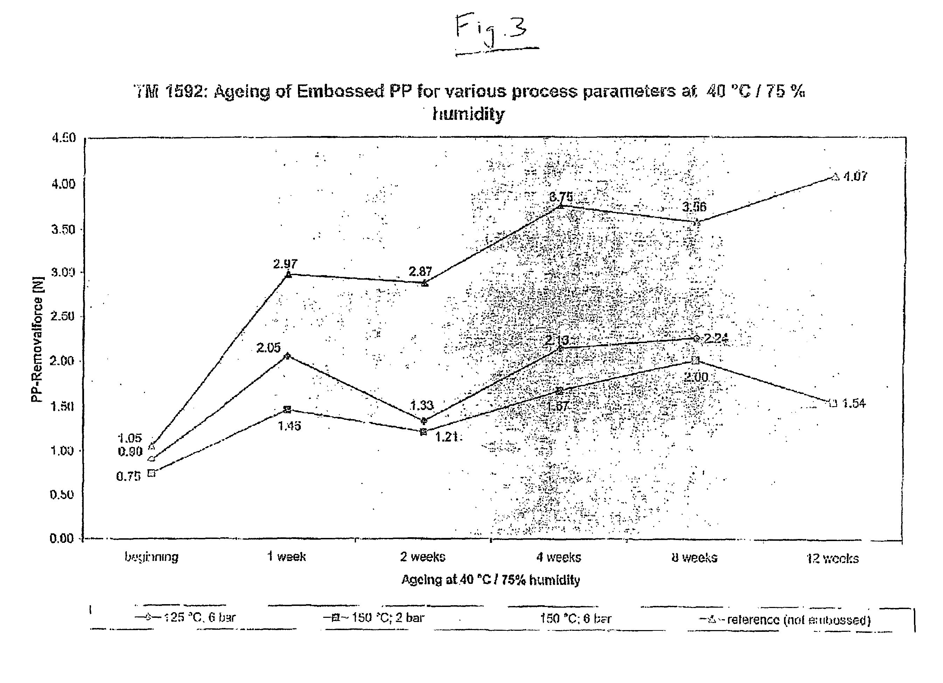

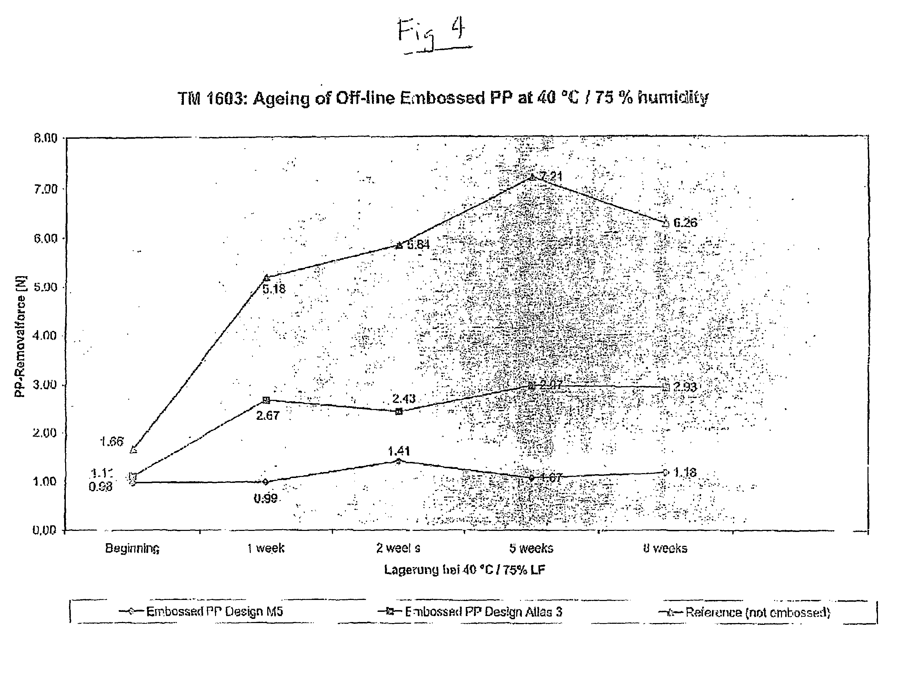

Data resulting from these tests are provided in Tables 1-4 and graphed in Figs. 1A-4. These data illustrate that tampons packaged in useful overwrap packages can have acceptable removal forces.

**Table 1**

| PP-Removalforce [N] | | | | | | | |
|---|---|---|---|---|---|---|---|
| | | Beginning | 1 week | 2 weeks | 4 weeks | 9 weeks | 12 weeks |
| String end | | 0.64 | 0.51 | 1.39 | 0.63 | 0.76 | 1.10 |
| *Stdev* | | *0.38* | *0.34* | *1.72* | *0.36* | *0.41* | *0.55* |

**Table 2**

| PP-Removalforce [N] | | | | | | | |
|---|---|---|---|---|---|---|---|
| | | Beginning | 1 week | 2 weeks | 4 weeks | 8 weeks | 12 weeks |
| Tampons with Embossed PP | | 0.79 | 1.28 | 1.15 | 1.06 | 1.29 | 0.94 |
| | *Stdev* | *0.57* | *0.71* | *0.62* | *0.76* | *0.63* | *0.58* |
| Tampons with Standard PP | | 1.91 | 5.02 | 5.99 | 7.62 | 8.43 | 7.58 |
| | *Stdev* | *0.33* | *0.93* | *1.20* | *1.80* | *2.00* | *1.67* |

**Table 3**

| **40°C / 75 % LF** | | Ageing of Embossed PP for various process parameters | | | | | |
|---|---|---|---|---|---|---|---|
| PP-Removalforce [N] | | | | | | | |
| Sealing with | | beginning | 1 week | 2 weeks | 4 weeks | 8 weeks | 12 weeks |
| 125 °C; 6 bar | | 0.90 | 2.05 | 1.33 | 2.13 | 2.24 | |
| | *Stdev* | *0.21* | *0.64* | *0.36* | *0.95* | *0.88* | |
| 150 °C; 2 bar | | 0.75 | 1.46 | 1.21 | 1.67 | 2.00 | 1.54 |
| | *Stdev* | *0.15* | *0.39* | *0.44* | *0.79* | *0.70* | *0.78* |
| 150 °C; 6 bar | | 0.84 | 1.61 | 1.10 | 1.03 | 0.97 | 0.96 |
| | *Stdev* | *0.16* | *0.53* | *0.34* | *0.23* | *0.26* | *0.38* |
| reference (not embossed) | | 1.05 | 2.97 | 2.87 | 3.75 | 3.56 | 4.07 |
| | *Stdev* | *0.24* | *0.66* | *0.60* | *1.08* | *0.69* | *1.40* |

**Table 4**

| Ageing of Off-line | | | | | | |
|---|---|---|---|---|---|---|
| **40°C / 75 % LF** Embossings | | | | | | |
| PP-Removalforce [N] | | | | | | |
| | Beginning1 | | week2 | weeks 5 | weeks | 8 weeks |
| Embossed PP | | | | | | |
| Design M5 | | 0.98 | 0.99 | 1.41 | 1.07 | 1.18 |
| | *Stdev* | *0.22* | *0.18* | *0.48* | *0.21* | *0.30* |

| Embossed PP | | | | | | |
|---|---|---|---|---|---|---|
| Design Atlas 3 | | 1.11 | 2.67 | 2.43 | 2.97 | 2.93 |
| | *Stdev* | *0.34* | *1.12* | *0.69* | *1.06* | *0.90* |
| Reference (not embossed) | | 1.66 | 5.18 | 5.84 | 7.21 | 6.26 |
| | *Stdev* | *0.48* | *1.40* | *1.36* | *1.33* | *0.98* |

The specification and embodiments above are presented to aid in the complete and non-limiting understanding of the invention disclosed herein. Since many variations and embodiments of the invention can be made without departing from its scope, the invention resides in the claims hereinafter appended.

## Claims

1. A packaged tampon useful for feminine hygiene comprising:
a) a compressed tampon having an elongate absorbent structure;
b) an apertured polymeric film cover substantially surrounding the absorbent structure; and
c) an overwrap package comprising a polymeric film in contact with the apertured polymeric film cover and containing the tampon under compression;
wherein the overwrap package is removable from the compressed tampon by sliding it along at least a portion of a length of the tampon by applying a shear force of less than 5 Newtons between the package and the tampon after conditioning at 40°C/75 % humidity for one week, **characterized in that**
the polymeric film of the overwrap package has at least one embossed surface, and the at least one embossed surface is in contact with the apertured polymeric film cover of the tampon or has at least one corona-treated surface, and the at least one corona-treated surface is in contact with the apertured polymeric film cover of the tampon, or
wherein the film from which the tampon cover is formed is embossed or corona-treated on the surface which is in contact with the polymeric film of the overwrap package.

2. The packaged tampon of claim 1 wherein the overwrap package is removable from the compressed tampon by sliding it along at least a portion of a length of the tampon by applying a shear force of less than 3 Newtons between the package and the tampon after conditioning at 40°C/75 % humidity for one week.

3. The packaged tampon of claim 1 or 2 wherein the at least one embossed surface is microembossed.

4. The packaged tampon of claim 1, wherein the pattern embossed on the overwrap package film takes the form of regular array of pyramids having a height of less than 100 µm.

5. The packaged tampon of claim 1 further comprising a slip agent disposed on at least one of the apertured polymeric film cover and the overwrap package.

6. The packaged tampon of claim 2 wherein the overwrap package is removable from the compressed tampon by sliding it along at least a portion of a length of the tampon by applying a shear force of less than 2 Newtons between the package and the tampon after conditioning at 40°C/75 % humidity for one week.

## Patentansprüche

1. Verpackter Tampon, der für die Frauenhygiene geeignet ist, umfassend;
a) einen komprimierten Tampon mit einer länglichen absorbierenden Struktur;
b) eine mit Öffnungen versehene polymere Folienhülle, welche die absorbierende Struktur im Wesentlichen umgibt;
und
c) eine Umhüllungsverpackung, die eine polymere Folie in Kontakt mit der mit Öffnungen versehenen polymeren Folienhülle umfasst und die den Tampon unter Kompression hält;
wobei die Umhüllungsverpackung vom komprimierten Tampon entfernbar ist, indem sie entlang zumindest eines Abschnitts einer Länge des Tampons geschoben wird, durch Anbringen einer Scherkraft von weniger als 5 Newton zwischen der Verpackung und dem Tampon nach Konditionieren bei 40° C/75 % Feuchtigkeit über eine Woche hinweg, **dadurch gekennzeichnet, dass**
die polymere Folie der Umhüllungsverpackung
zumindest eine geprägte Oberfläche aufweist und die zumindest eine geprägte Oberfläche in Kontakt mit der mit Öffnungen versehenen polymeren Folienhülle des Tampons steht oder
zumindest eine koronabehandelte Oberfläche aufweist und die zumindest eine koronabehandelte Oberfläche in Kontakt mit der mit Öffnungen versehenen polymeren Folienhülle des Tampons steht
oder wobei die Folie, aus welcher die Tamponhülle gebildet ist, geprägt oder koronabehandelt auf der Oberfläche ist, die in Kontakt mit der polymeren Folie der Umhüllungsverpackung steht.

2. Verpackter Tampon nach Anspruch 1, **dadurch gekennzeichnet, dass** die Umhüllungsverpackung vom komprimierten Tampon entfernbar ist, indem sie entlang zumindest eines Abschnitts einer Länge des Tampons geschoben wird, durch Aufbringen einer Scherkraft von weniger als 3 Newton zwischen der Verpackung und dem Tampon nach Konditionieren bei 40 °C / 75 % Feuchtigkeit über eine Woche hinweg.

3. Verpackter Tampon nach Anspruch 1 oder 2, **dadurch gekennzeichnet, dass** zumindest eine geprägte Oberfläche mikrogeprägt ist.

4. Verpackter Tampon nach Anspruch 1, **dadurch gekennzeichnet, dass** das auf der Umhüllungsverpackungsfolie geprägte Muster die Form einer regelmäßigen Anordnung von Pyramiden mit einer Höhe von weniger als 100 µm aufweist.

5. Verpackter Tampon nach Anspruch 1, welcher weiterhin ein Gleitmittel umfasst, welches auf der mit Öffnungen versehenen polymeren Folienhülle und/oder der Umhüllungsverpackung aufgebracht ist.

6. Verpackter Tampon nach Anspruch 2, **dadurch gekennzeichnet, dass** die Umhüllungsverpackung vom komprimierten Tampon entfernbar ist, indem sie entlang zumindest eines Abschnitts einer Länge des Tampons geschoben wird, durch Aufbringen einer Scherkraft von weniger als 2 Newton zwischen der Verpackung und dem Tampon nach Konditionieren bei 40° C/75 % Feuchtigkeit über eine Woche hinweg.

## Revendications

1. Tampon emballé utile pour l'hygiène féminine, comprenant :
a) un tampon comprimé ayant une structure absorbante allongée ;
b) une pellicule polymère perforée entourant substantiellement la structure absorbante ; et
c) un suremballage comprenant un film polymère en contact avec la pellicule polymère perforée et contenant le tampon comprimé ;
➢ le suremballage pouvant être retiré du tampon comprimé en le faisant glisser le long d'au moins une partie d'une longueur du tampon en appliquant une force de cisaillement inférieure à 5 Newton entre l'emballage et le tampon après conditionnement à 40°C/75 % d'humidité pendant une semaine, **caractérisé en ce que**
➢ le film polymère du suremballage comporte au moins une surface gaufrée et au moins la surface gaufrée est en contact avec la pellicule polymère perforée du tampon ou a au moins une surface traitée en couronne et au moins la surface traitée en couronne est en contact avec la pellicule polymère perforée du tampon, ou
➢ le film constituant la pellicule du tampon est gaufré ou a subi un traitement en couronne sur la surface qui est en contact avec le film polymère du suremballage.

2. Tampon emballé selon la revendication 1, dans lequel le suremballage peut être retiré du tampon comprimé en le faisant glisser le long d'au moins une partie d'une longueur du tampon en appliquant une force de cisaillement inférieure à 3 Newton entre l'emballage et le tampon après conditionnement à 40°C/75 % d'humidité pendant une semaine.

3. Tampon emballé selon la revendication 1 ou 2, dans lequel au moins une surface gaufrée présente un micro-gaufrage.

4. Tampon emballé selon la revendication 1, dans lequel la configuration gaufrée sur le film du suremballage prend la forme de séries régulières de pyramides ayant une hauteur inférieure à 100 µm.

5. Tampon emballé selon la revendication 1, comprenant en outre un agent glissant disposé sur au moins soit la pellicule polymère perforée, soit le suremballage.

6. Tampon emballé selon la revendication 2, le suremballage pouvant être retiré du tampon comprimé en le faisant glisser le long d'au moins une partie d'une longueur du tampon en appliquant une force de cisaillement inférieure à 2 Newton entre l'emballage et le tampon après conditionnement à 40°C/75 % d'humidité pendant une semaine.
